# EUROPÄISCHE PATENTSCHRIFT

(11) **EP 3 042 648 B1**
(45) Veröffentlichungstag und Bekanntmachung des Hinweises auf die Patenterteilung: **05.06.2019**
(21) Anmeldenummer: 15202259.6
(22) Anmeldetag: 23.12.2015
(51) Int. Cl.: A61K 9/16

(54) **DIREKTGRANULAT MIT RETARDIERTEM WIRKSTOFF**
DIRECT GRANULATE WITH RETARDED AGENT
GRANULES DIRECTS A AGENT ACTIF RETARDE

(30) Priorität: 23.12.2014 EP 14200223
(43) Veröffentlichungstag der Anmeldung: 13.07.2016
(73) Patentinhaber: Hermes Arzneimittel GmbH, 82049 Pullach (DE)
(72) Erfinder: DANDL, Klaus, 82041 Oberhaching (DE); HAALA, Josef, 82049 Pullach (DE)
(74) Vertreter: Pharma Patents International AG

(56) Entgegenhaltungen:
- EP-A2- 0 443 743
- FR-A- 1 601 721
- JP-A- H05 221 859
- JP-B2- 3 122 510

## Beschreibung

Die vorliegende Erfindung betrifft Direktgranulate, welche den leicht wasserlöslichen Wirkstoff Ascorbinsäure (Vitamin C) in retardierter Form enthalten. Die Zusammensetzungen werden unter anderem als Nahrungsergänzungsmittel und/oder in der Therapie oder Prävention von Vitamin C-Mangelzuständen eingesetzt.

### Hintergrund der Erfindung

Da der Mensch, anders als eine Mehrzahl der Tiere, Vitamin C nicht selbst aus Glukose zu synthetisieren vermag, muss dieses wichtige Vitamin im Allgemeinen durch externe Quellen substituiert werden, um in Phasen erhöhten Bedarfes eine ausreichende Vitamin C-Versorgung zu sichern; bspw. bei Stress, Mangelernährung, Infektionen, Allergien, chronisch entzündlichen Erkrankungen wie Arthritis und/oder bei Älteren. Wie bei vielen Wirkstoffen mit guter Wasserlöslichkeit (z. B. löslich, leicht löslich und sehr leicht löslich gemäß Definition des Europäischen Arzneibuchs) besteht für Vitamin C hierbei das Problem, dass der Körper überschüssiges Vitamin C - also solche Mengen, die er akut nicht verwenden oder in endogene Speicher einlagern kann - rasch mit dem Urin wieder ausgeschieden werden. Dies ist umso relevanter, da der Körper keine geeigneten Speicher für Vitamin C besitzt.

Um also eine anhaltende, möglichst konstante Vitamin C-Versorgung des Körpers zu gewährleisten, wurden bereits Formulierungen mit kontrollierter Freisetzung entwickelt; beispielsweise retardierte Freisetzung (verzögerte Freisetzung) oder pulsatile Freisetzung (d.h. in mehreren "Pulsen" über einen längeren Zeitraum).

So beschreibt z. B. die internationale Patentanmeldung WO 00/69420 A1 eine Arzneiform bei der das Vitamin C im Magendarmtrakt in drei Freisetzungs-Pulsen freigesetzt wird. Hierzu werden herkömmliche, marktübliche Vitamin C Partikel gesiebt und die Fraktion größer als 200 µm dann mittels Sprühverfahren mit verschiedenen Polymerüberzügen versehen, um sog. Mikrokapseln zu erhalten. Durch Mischen von drei verschiedene Chargen dieser Vitamin C-Mikrokapseln (eine überzugsfreie, eine mit einem Hypromellose-Phthalat-Überzug, sowie eine mit einem Eudragit®-Überzug), lassen sich drei Freisetzungs-Pulse erreichen, deren Zeitpunkt unter anderem davon auch davon abhängt in welchen Anteilen die drei Chargen gemischt werden.

Alternativ zu gepulster Freisetzung, ist auch eine zeitlich retardierte Freisetzung für Vitamin C bekannt; z. B. sogenannte Langzeit-Vitamine wie das marktübliche "Cetebe Vitamin C Retard 500". Hierbei handelt es sich eine Kapselformulierung, befüllt mit überzogenen Sucrose-Pellets (sog. Non-Pareils), die in jeweils alternierenden Schichten Vitamin C sowie Schellack enthalten. Da sich Schellack im menschlichen Magensaft nicht auflöst (Schellack ist ein sog. enterisches Polymer), wird das Polymer erst im Darm Schicht um Schicht aufgelöst, bzw. aufgequollen, und dadurch das Vitamin C allmählich über einen Zeitraum von ca. 6-9 h in kleinen Portionen freigesetzt.

WO 00/54754 A2 schlägt die verzögerte Freigabe von Vitamin C aus Ascorbinsäureteilchen vor, die mit hydrophoben Überzugsmaterialien wie Bienenwachs, Glycerolmonostearat, Stearinsäure, Cetylstearylalkohol oder Silikonen in verschiedener Dicke überzogen sind. Es können auch Mischungen solcher Teilchen verwendet werden. Die überzogenen Ascorbinsäureteilchen sind zweckmäßiger Weise in einer üblichen Gelatinekapsel zur oralen Verabreichung vorgesehen.

Leider bergen solche Kapsel-Formulierungen diverse Nachteile für den Anwender. Die Kapseln werden häufig aus Rinder- oder Schweinegelatine gefertigt, was sowohl für einige religiöse Gruppen problematisch ist, als auch für Vegetarier oder Veganer. Darüber hinaus ist zum Schlucken von Kapseln immer eine ausreichende Menge Flüssigkeit nötig. Viele Menschen haben zudem Probleme mit dem Schlucken von Kapseln, da diese auf Flüssigkeiten aufschwimmen (z. B. auch im Mund beim Trinken), leicht an der Mund-und Rachenschleimhaut anhaften können und aufgrund der fehlenden Komprimierung und der eingeschlossenen Luft meist größer sind als Tabletten mit vergleichbarer Dosis.

Letzteres Problem ist besonders ausgeprägt für das leicht lösliche Vitamin C, bei dem für hohe Einzeldosen von üblicherweise ca. 300 bis 1000 mg eine kontrollierte Freisetzung angestrebt wird. Es ist dem Fachmann hinreichend bekannt, dass üblicherweise Hilfsstoffe vonnöten sind, um kontrollierte Freisetzung zu erreichen; und im Allgemeinen bedarf es hierbei umso mehr Hilfsstoffe, je höher die Löslichkeit des Wirkstoffes ist. Auf diese Weise kann sich das Gesamtgewicht der fertigen Formulierung für einen Wirkstoff wie Vitamin C (Retarddosis z. B. 500 mg; Wasserlöslichkeit_{20°C} = 333 mg/mL) schnell im Bereich von 0,5 bis 2 g bewegen; ein Gewicht, das sowohl in Tabletten- als auch Kapselform groß genug ist, um deutliche Beschwerden bei der oralen Anwendung nach sich zu ziehen.

Es ist zwar erlaubt, z. B. die Cetebe-Kapseln zu öffnen und die enthaltenen Pellets ohne die Kapselumhüllung einzunehmen. Allerdings sind die Schellackumhüllten Pellets immer noch ausreichend groß, um als körnig-kugelige Fremdkörper im Mund wahrgenommen zu werden; noch verstärkt dadurch, dass sie kein angenehmes Mundgefühl bieten, und sehr hart und glatt sind. Dies wiederum verleitet Anwender dazu, die Pellets (zer)kauen zu wollen; insbesondere dann, wenn eine Einnahme ohne Flüssigkeiten nötig oder gewünscht ist, z. B. weil der Anwender unterwegs kein Wasser bei sich hat. Ein solches Zerkauen würde die retardierte Freisetzung beeinflussen oder gar verhindern. Zudem würde ein nicht unerheblicher Teil der Vitamin C-Tagesdosis von 500 mg dann bereits im Mund frei, was aufgrund des Säurecharakters von Vitamin C zu stark saurem, unangenehmem Geschmack und im schlimmsten Fall zu Schäden an den Zähnen führen kann.

Weitere überzogene Vitamin C Formulierungen sind aus der Futtermittel- und Nahrungsmittel-Industrie bekannt. Hierbei werden die Überzüge aber vorrangig zur Stabilisierung des Vitamin C eingesetzt, z. B. um das Vitamin C vor Einflüssen durch Feuchtigkeit, Sauerstoff, Metalle etc. zu schützen.

So beschreibt bspw. FR 1601721 A Vitamin C Partikel, welche mit Lipiden überzogen werden, vorzugsweise Hartfette mit einem Schmelzpunkt (Mₚ) zwischen 35 °C und 80 °C, z. B. Talg (Mₚ 38-40 °C), Kakaobutter (Mₚ 36-38 °C) oder gehärtetes Rizinusöl (Mₚ 79-80 °C). Eine kontrollierte Freisetzung wird hierbei nicht angestrebt; vielmehr betont FR 1601721 A, dass Lipidüberzüge im Gegensatz zu sonst üblichen Polymerüberzügen die Absorption des Vitamin C nicht behindern, da die Lipide im Magen-Darm-Trakt einfach verdaut würden. Darüber hinaus scheint das erhaltene lipid-überzogene Produkt nur bedingt zur direkten oralen Verabreichung geeignet, da der Anteil an überzogenem Vitamin C in essbaren Mischungen stets sehr gering gewählt wird (bspw. 1 % in Glucose-Tabletten, 0,2 % in Milchpulver oder 0.05 % in Hennen-Futtermischung).

JPH05221859A beschreibt Vitamin C mit mehrfachen Überzügen. Die Vitamin C-Partikel (bevorzugt vorgranuliert zu runden Partikeln von ca. 400-800 µm Größe) werden in einem Dragierkessel mit einer ersten Schmelze eines Lipids oder Lipidgemisches überzogen (z. B. gehärtetes Sojaöl, Rindertalg, Mono-di-und triglyceride, aber auch Wachse). Nach dem Abkühlen folgt in der Wirbelschicht ein Pulverbeschichtungs-Schritt mit essbaren Materialien (z. B. Eiweißpulver), in dessen Anschluss aus alkoholischer Lösung eine Polymerschicht aufgebracht wird (z. B. Zein oder Schellack). Zuletzt folgt eine zweite Lipidschicht; erneut im Dragierkessel aufgebracht und optional aus demselben Material bestehend wie die erste Lipidschicht. Das so erhaltene überzogene Vitamin C Produkt wird anschließend als Vitaminzusatz zu Viehfuttermischungen zugesetzt und weist dabei eine größere Stabilität auf als nicht überzogenes Vitamin C. Es ist aber anzunehmen, dass das vierfach umhüllte Vitamin C mit einer Partikelgröße von ca. 400 - 1000 µm ein Fremdkörpergefühl im Mund erzeugt und somit bei direkter oraler Gabe zum Zerkauen verleiten würde.

EP 0443743 A2 offenbart ebenfalls Lipidüberzogenes Vitamin C, welches als stabilisierter Vitaminzusatz für Viehfutter bzw. Fischfutter gedacht ist (ca. 0,0001 - 5 % Vitamin C Gehalt). Die Vitamin C-Partikel werden mit einer pulverförmigen Mischung aus Vitamin E und einem Lipid mit einem Schmelzpunkt von mindestens 40 °C (bspw. gehärtetes Raps- oder Sojaöl) beschichtet, wobei der Schmelzpunkt so gewählt ist, dass eine Agglomeration der überzogenen Vitamin C-Partikel während der Lagerung bei Raumtemperatur vermieden wird. Optional kann das Vitamin C vorgranuliert werden, bspw. mit Hilfe von Zucker-oder Proteinlösungen. Die Lipid-überzogenen Vitamin C-Partikel erzielen eine größere Stabilität des Vitamin C in Futtermischungen sowie eine leichte/hohe Resorbierbarkeit.

Der vorliegenden Erfindung liegt somit die Aufgabe zugrunde, eine alternative retardierte Zusammensetzung für das leicht wasserlösliche Vitamin C zur Verfügung zu stellen. Die Zusammensetzung sollte leichter und angenehmer verabreichbar sein als gängige Tabletten oder Kapseln mit retardiertem Vitamin C, bevorzugt auch ohne zusätzliche Flüssigkeitsgabe. Sie sollte zudem die Stabilität des Vitamin C gewährleisten und einfach und wirtschaftlich herstellbar sein.

Diese Aufgabe wird erreicht durch die erfindungsgemäße feste pharmazeutische Zusammensetzung in Form eines oralen Direktgranulats mit retardierter Wirkstofffreisetzung gemäß Anspruch 1, welches enthält:
(a) Retardpartikel mit einem Kern aus kristalliner Ascorbinsäure und einem Überzug aus einem Lipid mit einem Schmelzpunkt von mindestens 50 °C und optional einem oder mehreren weiteren Hilfsstoffen, wobei das Gewichtsverhältnis zwischen Kern und Überzug zwischen 50:50 und 90:10 liegt; und
(b) einen oder mehrere wasserlösliche Hilfsstoffe ausgewählt aus der Gruppe der Zucker, Zuckeralkohole und Oligosaccharide; sowie
(c) optional einen oder mehrere weitere Hilfsstoffe,
wobei Hilfsstoffe gemäß (b) oder (c) optional in granulierter Form vorliegen.

Im engen Sinne sind Granulate sogenannte Haufwerke, die mittels Aggregation oder Agglomeration von Pudern und/oder Pulvern hergestellt werden und somit meist gröber und körniger als Puder und Pulver sind, wobei allerdings die Grenzen der Partikelgrößen insbesondere von Pulvern und Granulaten häufig überlappen. Gängige Verfahren zur Granulierung bzw. Aggregation sind dem Fachmann bekannt und in der Literatur beschrieben.

Der Begriff "orale Direktgranulate" dagegen wird vom Fachmann deutlich breiter verstanden. Anders als bei den Granulaten im engen Sinne ist es bei oralen Direktgranulaten meist nicht von Belang, ob das Haufwerk tatsächlich durch eine Aggregation von Pulverpartikeln gewonnen wurde, oder ob ein oder mehrere Bestandteile des Direktgranulates anderweitig verarbeitet wurden. Es handelt sich bei oralen Direktgranulaten primär um oral verabreichbare und in der Mehrzahl fließfähige Haufwerke, welche direkt, also in ihrer granulären bzw. partikulären, Form angewendet werden. Beispielsweise können sie in den Mund und/oder auf die Zunge gestreut und dann leicht geschluckt werden.

Der Vorteil des erfindungsgemäßen Direktgranulates liegt darin, dass es üblicherweise ohne zusätzliche Flüssigkeit, allein mittels des im Mund vorhandenen Speichel durchfeuchtet bzw. suspendiert und dann leicht und angenehm geschluckt werden kann, dabei aber gleichzeitig die retardierte Freisetzung des leicht löslichen Vitamin C sicherstellt, selbst wenn dieses in höheren Dosen von z. B. 500 mg vorliegt. Letzteres gelingt sonst häufig nur in Form voluminöser, schwer schluckbarer Kapsel- oder Tablettenformulierungen.

Diese vorteilhafte Schluckbarkeit wird u.a. durch eine fein aufeinander abgestimmte Selektion und Kombination von Parametern erreicht, wie z. B. der Größe der Retardpartikel, deren Gewichtsanteil im finalen Direktgranulat, die Wahl der Überzugsmaterialien sowie die Art und Menge der Hilfsstoffe, welche den Retardpartikeln beigemengt werden.

Im Zusammenhang mit der Erfindung bezeichnet der Begriff "Retardpartikel" solche Partikel, die einen Wirkstoff und mindestens einen Hilfsstoff enthalten und die so aufgebaut sind, dass der Hilfsstoff eine langsame Freisetzung des Wirkstoffs bewirkt. Dazu kann der Wirkstoff in dem Hilfsstoff gelöst oder suspendiert sein (sog. Matrix-Systeme), oder es kann ein Wirkstoffpartikel mit einer Hilfsstoffschicht umhüllt vorliegen (sog. Reservoir-Systeme).

Die erfindungsgemäße Zusammensetzung enthält Retardpartikel mit einem Kern aus kristalliner Ascorbinsäure und einem Überzug aus einem Lipid und optional einem oder mehreren weiteren Hilfsstoffen. Das Lipid hat einen Schmelzpunkt von mindestens 50 °C. Für den Fall, dass der Überzug ein weiteres Lipid enthält, hat mindestens eines der Lipide einen Schmelzpunkt von 50 °C. Im nicht unüblichen Fall, dass das Lipid keinen scharfen Schmelzunkt, sondern einen Schmelzbereich aufweist, ist unter dem Schmelzpunkt im Sinne der Erfindung die Untergrenze des Schmelzbereichs zu verstehen, also die Temperatur, bei der das Lipid während des Aufheizens zu schmelzen beginnt.

Der Lipidüberzug kann einen oder mehrere weitere Hilfsstoffe enthalten, mit denen seine Eigenschaften hinsichtlich Verarbeitbarkeit, Geschmack, Aussehen, Stabilität oder Wirkstofffreisetzung angepasst werden.

Erfindungsgemäß verwendet werden Überzüge mit mindestens 80 Gew.-% Lipid, mit mindestens 90 Gew.-%, oder solche, die praktisch ausschließlich aus Lipid bestehen.

Das Gewichtsverhältnis zwischen Kern und Überzug liegt erfindungsgemäß zwischen 50:50 und 90:10.

Der Lipidüberzug auf den Retardpartikeln stellt vorrangig die gewünschte retardierte Freisetzung des Wirkstoffs sicher, fungiert aber auch als Geschmacksmaskierung für das Vitamin C, da aufgrund der Retardierung kein oder kaum Vitamin C auf der Zunge freigesetzt wird.

Die Erfinder haben überraschenderweise festgestellt, dass sich insbesondere Lipide mit einem höheren Schmelzpunkt von mindestens 50 °C, bevorzugt mindestens 60 °C, hervorragend für orale Direktgranulate eignen. Diese Lipide sind zum einen ausreichend fest, um den überzogenen Wirkstoff stabil zu umhüllen und werden bei Körpertemperatur noch nicht (an)geschmolzen. Im Unterschied zu Lipiden mit Schmelzpunkten unter 50 °C (bspw. Trilaurin; Mₚ ca. 44-47 °C oder Rindertalg Mₚ ca. 40-45 °C) und den in WO 00/54754 A2 vorgeschlagenen hydrophoben Umhüllungen aus Wachsen oder Silikonen hinterlassen die erfindungsgemäßen Lipide zudem keinen seifigen oder fettigen (Geschmacks)Eindruck im Mund.

Gleichzeitig werden die erfindungsgemäßen Lipidüberzüge im Vergleich mit konventionellen Polymer-Überzügen aus bspw. Celluloseethern in der Mundhöhle und/oder auf und unter der Zunge subjektiv als weniger hart, weniger "plastikartig" wahrgenommen; erzeugen also weniger Fremdkörpergefühl. All dies trägt maßgeblich zu dem gewünschten angenehmen Mundgefühl des erfinderischen oralen Direktgranulates bei.

Das Mundgefühl wird u.a. durch strukturelle Eigenschaften mitbestimmt (also taktile Wahrnehmungen der Textur wie z. B. fettig, trocken, hart, formbar, klebrig, krümelig, etc.), sowie durch geometrische Eigenschaften (groß, klein, rund, kantig o.ä.) und des Weiteren durch Eigenschaften, die mit dem im Mund-und Rachenraum wahrgenommenen Wassergehalt (sahnig, saftig, trocken, spröde, o.ä.), Temperaturempfindungen (wärmend, kühlend) oder der Reizung freier Nervenenden (scharf, brennend, prickelnd) zusammenhängen.

Neben den Retardpartikeln enthält das orale Direktgranulat zumindest einen wasserlöslichen Hilfsstoff gemäß (b), mit dem die Retardpartikel vermischt werden. Es handelt sich hierbei erfindungsgemäß um Hilfsstoffe, die gemäß Definition des Europäischen Arzneibuchs löslich, leicht löslich oder sehr leicht löslich sind und die aus der Gruppe der Zucker, Zuckeralkohole und Oligosaccharide ausgewählt sind.

Optional können dem oralen Direktgranulat noch weitere Hilfsstoffe gemäß (c) zugesetzt werden, die beispielsweise die Viskosität der sich in situ bildenden Retardpartikel-Suspension leicht erhöhen, den Speichelfluss anregen, den Geschmack beeinflussen oder die Riesel- bzw. Fließfähigkeit des Granulates regulieren. Optional können auch die Hilfsstoffe gemäß (c) wasserlöslich sein (also löslich, leicht löslich oder sehr leicht löslich gemäß der Definition des Europäischen Arzneibuchs).

Weiterhin optional können einzelne oder alle der Hilfsstoffe, welche den Retardpartikeln beigemischt werden, zuvor granuliert bzw. aggregiert werden. Dies kann z. B. ratsam sein, um einen potentiell störenden Feinstaubanteil zu reduzieren und/oder wenn die Partikelgröße(n) der Hilfsstoffe deutlich unter der Partikelgröße der Retardpartikel liegt und Entmischungen der verschiedenen Partikelfraktionen zu befürchten sind.

Um eine ausreichende Vitamin C-Versorgung über den Tag sicherzustellen, sollte die Retardierung über ca. 8-10 h andauern. Längere Retardierungen durch die Retardpartikel sind nicht mehr zielführend, da sich die Partikel nach 8-10 h normalerweise im Dickdarm befinden, wo sowohl die Resorptionsoberfläche als auch das für die Freisetzung des Vitamin C benötigte Wasser deutlich reduziert sind.

Das Freisetzungsprofil lässt sich z. B. durch die Zusammensetzung des Lipidüberzugs, oder über dessen Stärke (oder Dicke) steuern. Die Stärke ergibt sich wiederum aus der Auftragsmenge des Überzugsmaterials und aus der Oberfläche der zu überziehenden Partikel. Je nach der Größe der Vitamin C-Partikel und der sich daraus ergebenden Oberfläche ist demnach die Auftragsmenge des Überzugsmaterials zu wählen, mit dem die gewünschte Retardierung erzielt wird.

*In vitro* lässt sich das Freisetzungsprofil gemäß den gängigen Arzneibüchern mit einer genormten Freisetzungsapparatur unter standardisierten Bedingungen bestimmen. Erfindungsgemäß wird das Freisetzungsprofil bevorzugt in einer Freisetzungs-Apparatur mit Blattrührer (USP Dissolution Apparatus 2) in 900 mL demineralisiertem Wasser bei 37 °C bei einer Rührgeschwindigkeit von 75 UPM bestimmt. Unter diesen Bedingungen zeigt die Zusammensetzung eine Retardierung über mindestens etwa 4 bis 6 Stunden, vorzugsweise über mindestens etwa 6 bis 8 Stunden. Das heißt, vor Ablauf des jeweiligen Zeitraums ist der Wirkstoff noch nicht vollständig freigesetzt.

Bevorzugt sind Ausführungsformen, bei denen die Stärke des Lipidüberzugs auf den Retardpartikeln so gewählt ist, dass die Zusammensetzung innerhalb von 4 Stunden nicht mehr als 70 % der in ihr enthaltenen Ascorbinsäure freisetzt, gemessen unter den angegeben Bedingungen. Ebenso bevorzugt sind Ausführungen, welche nach 4 Stunden zwischen 40 % und 70 % des Wirkstoffs freigesetzt haben, und/oder nach 6 Stunden zwischen 50 % und 90 %, und/oder nach 8 Stunden zwischen 60 % und 100 %.

Um die gewünschte Retardierung über ca. 8-10 h und/oder von nicht mehr als 70 % innerhalb von 4 Stunden mit den Retardpartikeln zu erzielen, sollte das Gewichtsverhältnis zwischen Kern und Lipidüberzug zwischen 50:50 und 90:10 liegen. Vorteilhaft bei gängigen Korngrößen des Vitamin C ist auch ein Gewichtsverhältnis zwischen 60:40 und 80:20, z. B. 65:35, 70:30 oder 75:25. Wie bereits erwähnt, ist zu beachten, dass sich die benötigte Menge an Überzugsmaterial für ein und denselben Lipidüberzug ändern kann, wenn sich bspw. die Partikelform und -größe und/oder die Porosität des Überzugsgutes ändern.

Wie bereits erwähnt, lässt sich das Freisetzungsprofil nicht nur durch die Stärke (oder Dicke) des Lipidüberzugs steuern, sondern auch über dessen Zusammensetzung. Bevorzugt sind Ausführungsformen, bei denen die Zusammensetzung des Lipidüberzugs auf den Retardpartikeln so gewählt ist, dass die Zusammensetzung innerhalb von 4 Stunden nicht mehr als 70 % der in ihr enthaltenen Ascorbinsäure freisetzt, gemessen unter den oben genannten Bedingungen.

Bei den erfindungsgemäßen Retardpartikeln wird ein Lipidüberzug auf einen Kern aus kristalliner Ascorbinsäure aufgetragen, wobei das Lipid einen Schmelzpunkt von mindestens 50 °C aufweist. Lipide sind größtenteils wasserunlösliche bzw. hydrophobe Substanzen mit geringer Polarität. Zu den Lipiden gehören u.a. Fettsäuren, Triglyceride (Fette und fette Öle), Wachse, Phospholipide, Sphingolipide, Lipopolysaccharide und Isoprenoide. Vorzugsweise weist das Lipid einen Schmelzpunkt von mindestens 60 °C auf.

Es handelt sich bei den Lipiden im Überzug der Retardpartikel um Triglyceride (auch Triacylglycerole, Triacylglyceride, Glycerol-Triester oder seltener auch Neutralfette genannt). Sie sind dreifache Ester von Glycerol (Glycerin) als polarer, hydrophiler Kopfgruppe mit drei lipophilen Fettsäuremolekülen. In einer weiter bevorzugten Ausführungsform handelt es sich um Triglyceride natürlicher Fettsäuren. Beispiele solcher natürlicher Fettsäuren beinhalten Palmitinsäure oder Stearinsäure.

Innerhalb der Triglyceride gibt es sowohl einfache Formen (dreimal der gleiche Fettsäurereste an einem Glycerol-Molekül) als auch gemischte Formen (zwei oder drei verschiedene Fettsäurereste an einem Glycerol-Molekül). Häufig enthalten die Triglyceride zudem auch sog. Partialglyceride als Begleitstoffe; also Ester des Glycerols mit Fettsäuren, wobei einige Hydroxylgruppen des Glycerols unverestert sind. Erfindungsgemäß handelt es sich bei den Lipiden im Überzug um möglichst reine Formen einfacher Triglyceride, die mindestens 80 Gew.-% und bevorzugt mindestens 90 Gew.-% eines einfachen Triglycerids enthalten und nur wenig gemischte - und/oder Partialglyceride. Beispiele für solche einfachen Triglyceride sind Glyceroltripalmitat (Tripalmitoylglycerol), Glyceroltristearat (Tristearoylglycerol) oder Glyceroltriarachidat (Triarachidoylglycerol).

Optional können die Triglyceride auch miteinander gemischt werden.

Vorzugsweise weist das Lipid einen Schmelzpunkt von mindestens 60 °C auf. Insbesondere Glyceroltristearat mit einem Schmelzpunkt bei ca. 70-73 °C hat sich in Versuchen aufgrund seines höheren Schmelzpunktes als besonders angenehm im Mund herausgestellt; es hinterlässt deutlich weniger unangenehm seifige und/oder fettige (Geschmacks)Eindrücke als andere Triglyceride mit einem Schmelzpunkt von unter 50 °C, z. B. Rindertalg (Schmelzpunkt ca. 40 bis 45 °C) oder Trilaurin (Mₚ ca. 44 bis 47 °C). Dies ist insofern überraschend, als auch der Schmelzpunkt des Rindertalgs oder des Trilaurins deutlich oberhalb der Körpertemperatur liegt. Der Fachmann hätte somit angenommen, dass auch hier eine klare Eignung für orale Direktgranulate vorliegen würde.

Dies bedeutet ferner, dass nicht alle in der Literatur für Vitamin C Partikel beschriebenen Lipid-Überzüge per se geeignet sind, in einem oralen Direktgranulat Anwendung zu finden, auch wenn sie Schmelzpunkte oberhalb der Körpertemperatur aufweisen und/oder imstande sind, die chemische Stabilität der Ascorbinsäure zu erhöhen.

In einer speziellen Ausführungsform sind die Retardpartikel mit einem Überzug versehen, der zu mindestens 90 Gew.-% aus Glyceroltripalmitat und/oder Glyceroltristearat besteht; z. B. mit einem Überzug aus Dynasan®116 oder 118, oder Mischungen daraus. Weiterhin bevorzugt sind Retardpartikel mit einem Überzug, der weitgehend aus Glyceroltripalmitat und/oder Glyceroltristearat besteht, d.h. ohne weitere Hilfsstoffe; insbesondere ein Überzug der weitgehend aus Glyceroltristearat besteht.

Einer der Vorzüge von Überzügen, welche zu mindestens 90 Gew.-% aus Glyceroltripalmitat und/oder Glyceroltristearat bestehen (wie bspw. Dynasan®116 oder 118), ist, dass sie synthetisch gewonnen werden und häufig reiner sind als die Mehrzahl natürlicher oder partial-synthetischer Lipide; also z. B. weniger gemischte Triglyceride oder Partialglyceride aufweisen, und/oder klarer definierte Schmelzpunkte haben. Dies ist meist vorteilhaft in der Verarbeitung, wie später noch detaillierter ausgeführt wird.

Zudem ist bspw. Glyceroltristearat deutlich geschmacksneutraler als eine Vielzahl natürlicher oder partial-synthetischer Lipide. Es hinterlässt bspw. anders als Rindertalg (Mₚ ca. 40 bis 45 °C; bzw. selbst gehärtetes Rindertalg, Mₚ ca. 60 °C) keinen talg-typisch 'tierischen' oder 'deftigen' Geschmack auf der Zunge. Gehärtetes Rizinusöl (Mₚ 79-80 °C) ist ebenfalls aufgrund seines 'kratzenden' Geschmackseindrucks eher ungeeignet für orale Direktgranulate. Aber auch viele eher neutrale gehärtete Öle (wie gehärtetes Rapsöl oder gehärtetes Sojaöl) können unter Umständen in Direktgranulaten als geschmacklich unangenehm bzw. unpassend wahrgenommen werden und nicht mit frischen und/oder fruchtigen Aromen wie Orange, Kirsche, Minze, Beere, Tropenfrüchte oder dergleichen harmonieren.

Wie bereits erwähnt, können dem Lipidüberzug optional Hilfsstoffe zugefügt werden. Dies können z. B. wasserlösliche Substanzen sein, Substanzen mit einer geringeren Schmelztemperatur, quellbare Substanzen und/oder Netzmittel wie bspw. Tween®. Ein Ziel dieser Hilfsstoffe im Lipidüberzug ist es, die Stabilität des Filmes zu unterstützen und variablere Freisetzungsprofile zu ermöglichen. Beispiele für Hilfsstoffe, welche die Verarbeitbarkeit von Lipiden und Lipidmischungen potentiell verbessern können, sind Emulgatoren, vor allem solche mit mittlerem oder niedrigem HLB-Wert (hydrophilic-lipophilic balance) von etwa 12 oder weniger. Prinzipiell sollten hierbei natürlich nur solche Substanzen gewählt werden, die zum einen den Geschmackseindruck nicht negativ beeinflussen, und zum anderen keine Inkompatibilitätsrisiko mit weiteren Überzugsbestandteilen, im Speziellen mit dem/den Lipid(en), aufweisen (bspw. zu Phasentrennungen führen könnten); so eignet sich bspw. Tween®65 in Kombination mit Dynasan®116 oder 118. So kann ein geringer Zusatz eines derartigen Hilfsstoffs zur Einstellung des Freisetzungsprofils bei Lipidüberzügen mit hoher Schichtdicke dienen.

Aromen, Süßstoffe, Geschmackskorrigenzien, Farbstoffe usw. können verwendet werden, um die organoleptischen Eigenschaften der Retardpartikel anzupassen. Zur Verbesserung der Stabilität können z. B. ein oder mehrere Hilfsstoffe eingesetzt werden, die im Lipid eine gewünschte Kristallform induzieren oder stabilisieren. Die Freisetzung wiederum lässt sich potentiell durch die Einarbeitung eines hydrophilen oder amphiphilen Hilfsstoffes in den Überzug beschleunigen oder durch einen besonders lipophilen oder schwerlöslichen Hilfsstoff weiter retardieren.

In bevorzugten Ausführungsformen sind die Retardpartikel mit einem Überzug versehen, der zu mindestens 90 % aus Glyceroltripalmitat und/oder Glyceroltristearat besteht, bei dem das Gewichtsverhältnis zwischen Kern und Überzug zwischen 60:40 und 80:20 liegt. In einer ebenso bevorzugten Ausführungsform sind die Retardpartikel mit einem Überzug versehen, der zu mindestens 90 % aus Glyceroltristearat besteht, bei dem das Gewichtsverhältnis zwischen Kern und Überzug bei 70:30 liegt.

Die Partikelgröße von oralen Direktgranulaten sollte im Allgemeinen 600 µm nicht überschreiten, um ein Fremdkörpergefühl im Mund zu vermeiden und den Kaureflex gering zu halten. Mit anderen Worten, die Mehrzahl der Partikel passiert ein Sieb mit einer Maschenweite von ca. 600 µm, bevorzugt mindestens 80 % oder mindestens 90 %. In einer bevorzugten Ausführungsform der Erfindung haben die Retardpartikel eine gewichtsmittlere Teilchengröße zwischen 100 µm und 500 µm, z. B. zwischen 150 µm und 400 µm.

Die bis dato im Stand der Technik erwähnten Lipid-überzogenen Ascorbinsäure-Partikel liegen teilweise deutlich über dieser Teilchengröße und sind somit zur Verwendung in einem oralen Direktgranulat ungeeignet; siehe bspw. Vitamin C-Starterkerne mit Partikelgrößen von ca. 400 - 800 µm in JP H05221859 A. Hier sind die Partikel schon vor dem Auftragen der vier Überzugsschichten ausreichend groß, um ein deutliches Fremdkörpergefühl im Mund zu erzeugen. Dies würde bei direkter oraler Gabe wahrscheinlich zum zerkauen verleiten und somit die in der vorliegenden Erfindung beabsichtigte Retardierung des Vitamin C gefährden.

In einer weiter bevorzugten Ausführungsform der Erfindung haben auch die beigemengten Hilfsstoffe bzw. Hilfsstoffpartikel eine gewichtsmittlere Teilchengröße zwischen 100 µm und 500 µm.

Die erfindungsgemäße Zusammensetzung enthält neben den Retardpartikeln mindestens einen wasserlöslichen Hilfsstoff gemäß (b), wahlweise in Pulverform oder in aggregierter Form (d.h. als Granulat im engeren Sinn). Wie bereits erwähnt, sind diese wasserlöslichen Hilfsstoffe gemäß (b) ausgewählt aus der Gruppe der Zucker, Zuckeralkohole und Oligosaccharide. Bei der Verabreichung des erfindungsgemäßen oralen Direktgranulates fungieren die wasserlösliche Hilfsstoffe gemäß (b) quasi als äußere Phase einer schluckbaren, in situ gebildeten Suspension, mit deren Hilfe die Retardpartikel leichter geschluckt werden können. Bevorzugt werden daher schmackhafte, wasserlösliche Hilfsstoffe wie Zucker, Zuckeralkohole oder wasserlösliche Vertreter der Oligosaccharide gewählt. Dies unterstützt auch die Compliance bei einer dauerhaften Anwendung des Direktgranulates.

Zwar ist es prinzipiell möglich, die Retardpartikel allein, ohne weitere Hilfsstoffe zu verabreichen (ohne die retardierende Wirkung einzubüßen), allerdings würden sie sich in diesem Fall nicht so leicht und angenehm schlucken lassen, länger im Mund verbleiben und vom Anwender leicht zerkaut werden, wodurch der Retardeffekt aufgehoben würde.

In einer speziellen Ausführungsform sind die oralen Direktgranulate so zusammengesetzt, dass die Retardpartikel zwischen 30 und 70 Gew.-% der Zusammensetzung ausmachen. In einer weiterhin bevorzugten Ausführungsform, beträgt der Gewichtsanteil der Retardpartikel an der gesamten Zusammensetzung zwischen 40 und 50 %. Diese Menge an Hilfsstoffen, insbesondere überwiegend wasserlöslichen Hilfsstoffen, bewirkt einen angenehmen Geschmack und ein angenehmes Mundgefühl, und sie erleichtert das Schlucken einer Dosiseinheit der Zusammensetzung. Insbesondere wird der Speichelfluss angeregt, so dass ein ausreichendes Volumen an Suspensionsmedium erzeugt wird.

Andererseits sollte die Menge an Hilfsstoffen vorzugsweise so gewählt sein, dass die Gesamtmenge an oralem Direktgranulat pro Dosis nicht mehr als ca. 3 g beträgt, oder nicht mehr als etwa 2 g oder 2,5 g, da größere Pulver- bzw. Granulatmengen im Mund und auf der Zunge nur noch schwierig gleichmäßig zur durchfeuchten sind. Andernfalls kann eine Einzeldosis in zwei oder mehreren Teilen (z. B. 2-3 Sachets mit nicht mehr als ca. 3 g Direktgranulat) eingenommen werden.

Dies ist insofern überraschend, da im Stand der Technik (bspw. FR 1601721 A) nur essbare Mischungen beschrieben sind, in welchen der Anteil an Lipidüberzogenem Vitamin C nur um 1 % oder unterhalb davon gewählt ist (1 % in Glucose-Tabletten, 0,2 % in Milchpulver oder 0.05 % in Hennen-Futtermischung). Im erfindungsgemäßen oralen Direktgranulat ist es dagegen nicht nötig, derart große Mengen essbarer Bestandteile zuzusetzen, um das Lipid-überzogene Vitamin C oral konsumierbar zu machen.

Gemäß einer weiteren Ausführungsform sind die oralen Direktgranulate zudem so zusammengesetzt, dass die Hilfsstoffe gemäß (b) und (c) mindestens 60 Gew.-%, bevorzugt mindestens 75 Gew.-%, eines in Wasser löslichen Zuckers oder Zuckeralkohols enthalten. Besonders bevorzugt sind Ausführungsformen, in denen die oralen Direktgranulate so zusammengesetzt sind, dass diese Hilfsstoffe mindestens 85 Gew.-%, eines in Wasser löslichen Zuckers oder Zuckeralkohols enthalten. Für die spezielle Ausführungsform, wo nur ein einziger Hilfsstoff (wahlweise granuliert oder in Pulverform) mit den Retardpartikeln vermengt wird, bedeutet dies, dass dieser Hilfsstoff ein löslicher Zucker oder Zuckeralkohol ist.

Erfindungsgemäß können die in Wasser löslichen Zucker oder Zuckeralkohole z. B. ausgewählt werden aus Saccharose, Maltitol, Mannitol, Sorbitol, Xylitol, und Mischungen derselben. In einer speziellen Ausführungsform sind die oralen Direktgranulate so zusammengesetzt sind, dass die Hilfsstoffe, die neben den Retardpartikeln vorliegen, mindestens 85 Gew.-% Sorbitol, Xylitol oder eine Mischung aus Sorbitol und Xylitol enthalten. Ein Beispiel hierfür ist ein Direktgranulat, welches etwa gleiche Gewichtsanteile von Retardpartikeln und Hilfsstoffen enthält, wobei die Hilfsstoffe zu über 85 Gew.-% aus einer Sorbitol-Xylitol Mischung bestehen. In alternativen, ebenso beispielhaften Ausführungsformen sind die oralen Direktgranulate so zusammengesetzt sind, dass die Hilfsstoffe mindestens 90 Gew.-% Maltitol oder mindestens 90 Gew.-% Saccharose enthalten.

Durch hohe Gewichtsanteile der löslichen Zucker, Zuckeralkohole oder Oligosaccharide wird ein ausreichendes Volumen an flüssigem wohlschmeckendem Suspensionsmedium für die Retardpartikel im Mund des Anwenders gebildet und die Schluckbarkeit sowie die Compliance des Anwenders positiv beeinflusst.

Optional können weitere Hilfsstoffe gemäß (c) enthalten sein; auch diese wahlweise in Pulverform, in aggregierter Form oder in Mischungen aus Pulvern und Aggregaten.

In einer weiter bevorzugten Ausführungsform enthält die Zusammensetzung einen sauren Hilfsstoff, z. B. als Bestandteil der Hilfsstoffe gemäß (c). Bevorzugte saure Hilfsstoffe sind Zitronensäure, Weinsäure, Äpfelsäure, Bernsteinsäure, sowie saure Salze derselben, etwa Mononatriumcitrat. Die Erfinder haben festgestellt, dass der Einsatz dieser sauren Substanzen - vor allem in Kombination mit den löslichen Zuckern und Zuckeralkoholen in den bevorzugten Mengen - eine zusätzliche Anregung des Speichelflusses hervorruft, durch den die Retardpartikel optimal dispergiert werden und besonders leicht zu schlucken sind.

Optional können die oralen Direktgranulate so zusammengesetzt sein, dass die Hilfsstoffe Zitronensäure, Mononatriumcitrat und/oder Dinatriumcitrat enthalten, insbesondere Zitronensäure in Kombination mit Mononatriumcitrat.

Davon unabhängig können die oralen Direktgranulate auch Trimagnesiumdicitrat (auch als Magnesiumdicitrat bekannt) als nicht saures Salz der Zitronensäure enthalten.

Weiter optional können die oralen Direktgranulate so zusammengesetzt sein, dass die Hilfsstoffe viskositätserhöhende Substanzen enthalten, z. B. Celluloseether wie Carmellose-Natrium. Ihre Konzentration sollte so gewählt werden, dass sie einerseits das Mundgefühl des Direktgranulates verbessern, und die in situ daraus entstehende Retardpartikel-Suspension stabilisieren; andererseits aber nicht das Schlucken erschweren.

Weiter optional können die oralen Direktgranulate so zusammengesetzt sein, dass die Hilfsstoffe neben den löslichen Zuckern oder Zuckeralkoholen weitere Süßungsmittel bzw. Süßstoffe, bspw. Sucralose, und/oder Aromen (Zitrone, Orange, Tropenfrüchte, Contramarum etc.) enthalten. Gängige und verträgliche Süßungsmittel und Aromen sind dem Fachmann hinreichend bekannt, z. B. aus oralen pädiatrischen Formulierungen.

Weiter optional können die oralen Direktgranulate so zusammengesetzt sein, dass die Hilfsstoffe Gleitmittel enthalten; z. B. dem Fachmann bekannte Fließregulierungs- und Schmiermittel wie hochdisperses Siliciumdioxid, Magnesiumstearat und/oder Stearinsäure. Magnesiumstearat wird hierbei bevorzugt. Um einen seifigen, wachsartigen oder fettigen Eindruck im Mund zu vermeiden, sollten diese Gleitmittel nur in geringen Mengen verwendet werden; üblicherweise 0,5 % oder weniger, bevorzugt 0,25 % oder weniger. Diese Substanzen dienen dazu, das orale Direktgranulat streufähig bzw. fließ- oder rieselfähig zu machen, so dass es leicht abgefüllt und auch leicht wieder aus Verpackungen entnommen werden kann.

In einer weiteren optionalen Ausführungsform können die oralen Direktgranulate so zusammengesetzt sein, dass sie neben den überzogenen Ascorbinsäure-Retardpartikeln auch einen Anteil nicht retardierter, nicht überzogener Ascorbinsäure enthalten, welcher bspw. als Initialdosis fungieren kann. Üblicherweise ist die Menge an nicht retardierter, nicht überzogener Ascorbinsäure kleiner als die der überzogenen Ascorbinsäure-Retardpartikeln; wie etwa zwischen 100 mg und 300 mg nicht überzogener Ascorbinsäure neben 300 mg bis 700 mg Ascorbinsäure-Retardpartikeln, z. B. 200 mg Initialdosis plus 550 mg retardierte Ascorbinsäure. Die beiden Teildosen sollten hierbei so gewählt sein, dass sie in der Summe eine Tagesdosis von ca. 1000 mg Ascorbinsäure nach Möglichkeit nicht überschreiten. Zwar wird überschüssiges Vitamin C renal ausgeschieden, allerdings steigt ab Tagesdosen von 1000 mg das Risiko der Bildung von Calciumoxalat-Nierensteinen, so dass solche Dosen Ausnahmefällen vorbehalten bleiben.

Weiter optional können die oralen Direktgranulate so zusammengesetzt sein, dass sie zusätzlich zur Ascorbinsäure Zinkverbindungen enthalten, bspw. Zinkoxid, Zinkgluconat, Zinkorotat, Zinkaspartat, Histidin-Hemizink o.ä.. Eine solche Kombination von Zink mit Vitamin C kann u.a. vorteilhaft sein, wenn das Vitamin C Direktgranulat zur Stärkung der Immunabwehr vorgesehen ist.

In einer speziellen Ausführungsform sind die oralen Direktgranulate so zusammengesetzt, dass die Hilfsstoffe gemäß (b) und (c) insgesamt nicht mehr als 5 Gew.-% von in Wasser schwerlöslichen Hilfsstoffen enthalten; d.h. umgekehrt sollten rund 95 Gew.-% oder mehr der Hilfsstoffe löslich sein. Dies ist wünschenswert, um das Fremdkörpergefühl im Mund so gering wie möglich zu halten.

Um dem Anwender die Dosierung des Vitamin C zu erleichtern, wird das erfindungsgemäße orale Direktgranulat bevorzugt in Einzeldosen abgefüllt und verpackt. So können die Einzeldosen der Zusammensetzung bspw. in Stickpacks, Sachets, Glasampullen oder Plastikampullen verpackt sein. Stickpacks sind hierbei besonders bevorzugt, da sie leicht und bruchsicher transportiert werden können und auch beim Öffnen durch Aufreißen üblicherweise eine kleinere Schüttöffnung bieten - und somit leichter vollständig in den Mund geleert werden können - als die vergleichbar aufgebauten, aber meist breiter dimensionierten Sachets. Zudem bieten Stickpacks aufgrund ihrer Laminierung eine einfache Möglichkeit zum Schutz des Direktgranulates vor Licht und Sauerstoff und stabilisieren so das in den Retardpartikeln enthaltene Vitamin C zusätzlich.

In einer bevorzugten Ausführungsform der Erfindung wird ein orales Direktgranulat bereitgestellt, enthaltend:
Retardpartikel mit einem Kern aus kristalliner Ascorbinsäure und einem Überzug welcher zu mindestens 90 Gew.-% aus Glyceroltripalmitat und/oder Glyceroltristearat besteht, bspw. Dynasan®116 oder 118, wobei das Gewichtsverhältnis zwischen Kern und Überzug zwischen 60:40 und 80:20 liegt;
einen oder mehrere wasserlösliche Hilfsstoffe ausgewählt aus Saccharose, Maltitol, Mannitol, Sorbitol, Xylitol, und Mischungen derselben, wobei der Gewichtsanteil dieses einen oder mehreren wasserlöslichen Hilfsstoffs am Gesamtgewicht der Hilfsstoffe außerhalb der Retardpartikel mindestens 85 Gew.-% beträgt;
einen oder mehrere speichelanregende Hilfsstoffe, ausgewählt aus Zitronensäure, Mononatriumcitrat und Mischungen derselben;
optional ein als Stabilisator agierendes Trockenmittel, z. B. Magnesiumdicitrat;
ein Gleitmittel ausgewählt aus Magnesiumstearat, Stearinsäure und hochdispersem Siliciumdioxid oder Mischungen derselben;
mindestens ein Aroma; sowie
optional weitere Süßstoffe oder Süßungsmittel,
wobei der Gewichtsanteil der Retardpartikel im oralen Direktgranulat zwischen 30 % und 70 % liegt.

In einer weiterhin bevorzugten Ausführungsform der Erfindung wird ein orales Direktgranulat bereitgestellt, enthaltend:
Retardpartikel mit einem Kern aus kristalliner Ascorbinsäure und einem Überzug bestehend aus Glyceroltristearat, wobei das Gewichtsverhältnis zwischen Kern und Überzug bei etwa 70:30 liegt;
einen oder mehrere wasserlösliche Hilfsstoffe ausgewählt aus Sorbitol, Xylitol und Mischungen derselben, wobei der Gewichtsanteil dieses einen oder mehreren wasserlöslichen Hilfsstoffs am Gesamtgewicht der Hilfsstoffe mindestens 85 % beträgt;
einen oder mehrere Speichel anregende Hilfsstoffe ausgewählt aus Zitronensäure, Mononatriumcitrat und Mischungen derselben;
optional ein als Stabilisator agierendes Trockenmittel, z. B. Magnesiumdicitrat;
ein Gleitmittel ausgewählt aus Magnesiumstearat, Stearinsäure und hochdispersem Siliciumdioxid oder Mischungen derselben;
mindestens ein Aroma-Hilfsstoff; sowie
Sucralose als weiteren Süßstoff,
wobei der Gewichtsanteil der Retardpartikel im oralen Direktgranulat zwischen 45 % und 55 % liegt.

In einem weiteren Aspekt der Erfindung wird ein Verfahren gemäß Anspruch 12, zur Herstellung der erfindungsgemäßen festen pharmazeutischen Zusammensetzung in Form eines oralen Direktgranulats bereitgestellt, enthaltend die folgenden Schritte:
(a) Bereitstellen von kristalliner Ascorbinsäure;
(b) Bereitstellen eines geschmolzenen Überzugsmaterials enthaltend ein Lipid mit einem Schmelzpunkt von mindestens 50 °C und optional einen oder mehrere weitere Hilfsstoffe;
(c) Fluidisieren der kristallinen Ascorbinsäure;
(d) Besprühen der fluidisierten kristallinen Ascorbinsäure mit dem geschmolzenen Überzugsmaterial;
(e) Abkühlen der überzogenen Ascorbinsäure, so dass das Lipid erstarrt und Retardpartikel erhalten werden; und
(f) Mischen der so erhaltenen Retardpartikel mit einem oder mehreren wasserlöslichen Hilfsstoffen und optional weiteren Hilfsstoffen.

Das in den Schritten (a) bis (e) beschriebene Verfahren kann in Wirbelschichtgeräten (sog. fluid-bed coater oder air-flow-bed coater) jeglicher Art durchgeführt werden, sofern die Geräte eine ausreichende Temperierung erlauben, um eine vorzeitige Erstarrung des Überzugsmaterials zu vermeiden. Die Sprührate der Schmelze sollte in jedem Fall so angepasst sein, dass eine gleichmäßige Befilmung gewährleistet ist, da größere Aggregate für ein gutes Mundgefühl des Direktgranulates nicht erwünscht sind.

Marktüblich verfügbare kristalline Ascorbinsäure hat im Allgemeinen bereits eine gewichtsmittlere Partikelgröße zwischen ca. 100 bis 300 µm und ist somit gut geeignet für das erfindungsgemäße orale Direktgranulat sowie für das beschriebene Herstellungsverfahren. Optional kann das Rohmaterial vor dem Fluidisieren und Besprühen gesiebt werden, um die bevorzugte Partikelgröße von 100 bis 300 µm sicherzustellen.

Nach dem Besprühen sollten die überzogenen Ascorbinsäurekristalle ausreichend lange abkühlen, um eine vollständige Erstarrung des Lipidüberzuges zu erlauben, bevor die so gewonnenen Retardpartikel mit den weiteren Hilfsstoffe des oralen Granulats gemischt und bspw. in Stickpacks abgefüllt werden.

Gemäß einer speziellen Ausführungsform liegt beim Besprühen das Gewichtsverhältnis zwischen der fluidisierten kristallinen Ascorbinsäure und dem geschmolzenen Überzugsmaterial zwischen 60:40 und 80:20; z. B. bei 65:35, 70:30 oder 75:25.

Vorzugsweise weist das Lipid einen Schmelzpunkt von mindestens 60 °C auf.

In einer bevorzugten Ausführungsform besteht das Überzugsmaterial zu mindestens 90 Gew.-% aus Glyceroltripalmitat und/oder Glyceroltristearat; z. B. aus Dynasan®116 oder 118.

In einer weiter bevorzugten Ausführungsform besteht der Überzug auf den kristallinen Ascorbinsäure-Partikeln aus Dynasan®118 und das Gewichtsverhältnis zwischen der fluidisierten kristallinen Ascorbinsäure und dem geschmolzenen Dynasan®118 liegt bei 70:30.

Der Schmelzpunkt von Dynasan®118 liegt bei ca. 70 bis 73 °C, daher sollte die Temperatur der zu sprühenden Schmelze hierbei einige Grad darüber liegen; z. B. bei 90 °C. Die Prozessluft-Temperatur beim Sprühen kann bspw. bei rund 30 bis 40 °C liegen, um eine gute Filmbildung auf den Ascorbinsäure-Kristallen zu gewährleisten.

In einigen Ausführungsformen kann sich - abhängig von der Auswahl des Überzugsmaterials - das Freisetzungsprofil im Laufe der Lagerzeit noch leicht verändern. Eine Konditionierung des Produkts bei erhöhter Temperatur, jedoch unterhalb der Schmelztemperatur des (oder der) im Überzugsmaterial eingesetzten Lipids (bzw. Lipide) kann vorteilhaft sein, um eine beschleunigte Einstellung des gewünschten Freisetzungsprofils zu erreichen. Eine solche Konditionierung ist insbesondere häufig dann von Nöten, wenn Überzüge mit variableren (Tri)glycerid-Zusammensetzung und/oder weiteren Schmelzbereichen verwendet werden, wie z. B. im Falle vieler natürlicher Lipide, aber auch partial-synthetischer Lipide. Häufig beruht die Veränderlichkeit der Freisetzung hierbei auf einer Umwandlung der Kristallstruktur von der α-Modifikation in die stabilere β-Modifikation während der Lagerung.

Einer der Vorzüge von Überzügen, welche zu mindestens 90 Gew.-% aus Glyceroltripalmitat und/oder Glyceroltristearat bestehen (wie bspw. Dynasan®116 oder 118), ist, dass diese synthetisch gewonnen werden und häufig reiner sind als die Mehrzahl natürlicher oder partial-synthetischer Lipide; also z. B. weniger gemischte Triglyceride oder Partialglyceride aufweisen, und/oder klarer definierte Schmelzpunkte haben. Dies kann bei der Verarbeitung hilfreich sein, da bspw. eine zu sprühende Schmelze nicht unnötig hoch erhitzt werden muss, um alle Bestandteile des Lipids vollständig aufzuschmelzen. Somit wird das Vitamin C beim Sprühen vor zu hohen Temperaturbelastungen geschützt und zugleich energie-effizient gearbeitet.

Bei Lipiden mit breiterem Schmelzbereich dagegen muss üblicherweise noch oberhalb des oberen Wertes dieses Schmelzbereichs gearbeitet werden, um eine homogene Zusammensetzung der Schmelze zu gewährleisten und ein mögliches Verstopfen der Sprühdüse(n) zu verhindern. Solche 'übererhitzten' Schmelzen härten auf den zu besprühenden Ascorbinsäure-Kristallen dann langsamer wieder aus, formen also den gewünschten Überzug weniger zügig. Zudem kann die Agglomerationstendenz durch das langsamere Aushärten erhöht sein. Weiterhin sind Entmischungen der Schmelze während des Erstarrens auf den zu besprühenden Ascorbinsäure-Kristallen möglich, wenn die höherschmelzenden Komponenten zuerst aushärten.

Darüber hinaus liegen die Überzüge mit mindestens 90 Gew.-% Glyceroltripalmitat und/oder Glyceroltristearat (wie bspw. Dynasan®116 oder 118) nach der Verarbeitung bereits in ihrer stabileren β-Modifikation vor, so dass sich die Freisetzung während der Lagerung nicht, bzw. nur marginal ändert. Die so erhaltenen Retardpartikel sind also deutlich lager-stabiler.

Die gemäß Verfahrensschritt (f) neben den Retardpartikeln vorliegenden Hilfsstoffe, werden üblicherweise zuerst homogen miteinander vermischt bevor sie mit den Retardpartikeln vermengt werden. Optional können einzelne oder alle dieser Hilfsstoffe vor dem Vermengen mit den Retardpartikeln granuliert bzw. aggregiert werden.

In einem weiteren Aspekt der Erfindung wird eine feste pharmazeutische Zusammensetzung in Form eines oralen Direktgranulats mit retardierter Wirkstofffreisetzung bereitgestellt, welche mittels des oben beschriebenen Verfahrens hergestellt wird.

### BEISPIELE

### Beispiel 1:

Marktübliche kristalline Ascorbinsäure-Partikel (Partikelgröße 100 bis 300 µm) werden in einem Hotmelt-Coating Verfahren mit geschmolzenem Dynasan®118 überzogen. Die Temperatur der Schmelze beträgt hierbei 78-80 °C, die Prozessluft-Temperatur im Wirbelschichtgerät liegt bei 90 °C.

Das Überzugslevel beträgt 42,9 % bezogen auf das Ursprungsgewicht der kristalline Ascorbinsäure-Partikel bzw. 30 % bezogen auf das Gewicht der überzogenen Ascorbinsäure-Partikel. 100 mg Retardpartikel enthalten 70 mg Ascorbinsäure und 30 mg des Dynasan®118.

### Beispiel 2:

Die in Beispiel 1 erhaltenen Ascorbinsäure-Retardpartikel werden mit verschiedenen Hilfsstoffmischungen versetzt; z. B. Hilfsstoffmischungen A, B und C gemäß Tabelle 1.

Die Hilfsstoffmischungen werden zuerst separat gemischt und danach den in Beispiel 1 erhaltenen Ascorbinsäure-Retardpartikeln zugemischt. Der Gewichtsanteil der überzogenen Ascorbinsäure-Retardpartikel am Gesamtgewicht der finalen Mischung, dem finalen oralen Direktgranulat, beträgt hierbei rund 48 % für die Granulate mit den Hilfsstoffmischungen A und B bzw. rund 51,0 % für die Hilfsstoffmischung C.

**Table 1: Zusammensetzung der Hilfsstoffpartikel-Mischungen (Angaben in %)**

| **Bestandteile** | **A** | **B** | **C** |
|---|---|---|---|
| Sorbitol | 61,05 | --- | --- |
| Xylitol | 25,45 | --- | --- |
| Maltitol | --- | 91,32 | --- |
| Saccharose | --- | --- | 90,30 |
| Sucralose | 0,13 | 0,15 | --- |
| Carmellose-Natrium | 1,02 | --- | --- |
| Zitronensäure | 3,82 | 3,18 | 3,65 |
| Mononatriumcitrat | 3,18 | 3,82 | 4,38 |
| Magnesiumdicitrat (wasserfrei) | 3,82 | --- | --- |
| Aroma | 1,27 | 1,27 | 1,46 |
| Magnesiumstearat | 0,25 | 02,5 | 0,22 |

### Beispiel 3: Freisetzungsverhalten

Das Freisetzungsverhalten von Vitamin C aus dem erfindungsgemäßen oralen Direktgranulat A nach mehrmonatiger Lagerung wurde mit dem von marktüblichen Cetebe® Vitamin C Retard 500-Kapseln verglichen. Hierfür wurden in einer arzneibuchgemäßen Freisetzungsapparatur mit Blattrührer (USP Dissolution Apparatus 2) eine Cetebe®-Kapsel (entsprechend 500 mg Vitamin C und beschwert bzw. fixiert in einem sog. Kapsel-Sinker) sowie eine dosisäquivalente Menge der überzogenen Ascorbinsäure-Partikel aus Beispiel 1 bei 37 °C in 900 mL deionisiertem Wasser freigesetzt. Die Rührgeschwindigkeit betrug 75 UPM. Alle Versuche wurden mindestens sechs Mal wiederholt und Mittelwerte sowie Standardabweichungen berechnet.

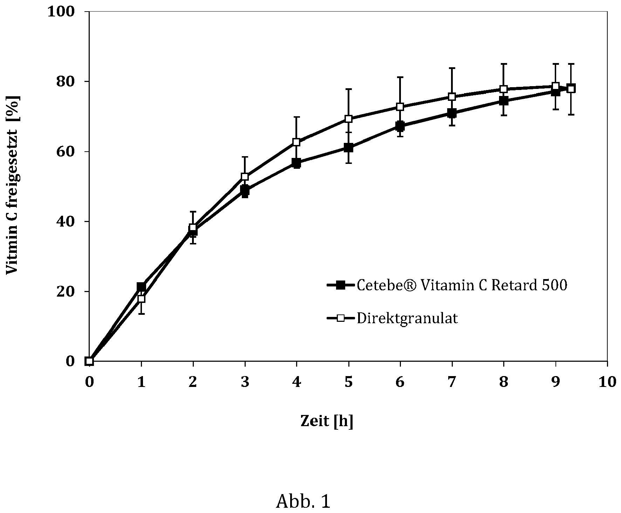

Wie aus Abbildung 1 ersichtlich, sind die Freisetzungsprofile der beiden Zusammensetzungen sehr ähnlich. Beide Zusammensetzungen gewährleisten die Retardierung über ca. 9 h.

### Beispiel 4: Zinkhaltige orale Direktgranulate

Um zinkhaltige orale Direktgranulate zu erhalten, wird zunächst eine zinkhaltige Vormischung hergestellt; z. B. ein Granulat aus 8,3 % Zinkoxid, 86,4 % Sorbitol und 5,3 % Miglyol® (Trigylceridgemisch aus Capryl-, Caprin- und Laurinsäure); entsprechend einem Gehalt von ca. 6.7 % Zink bezogen auf das Gewicht der Vormischung).

Diese zinkhaltige Vormischung wird anschließend mit den in Tabelle 2 aufgeführten Hilfsstoffen und den in Beispiel 1 erhaltenen Ascorbinsäure-Retardpartikeln gemischt, sowie optional mit einem Anteil nicht retardierter, also nicht überzogener Ascorbinsäure, welcher als Initialdosis fungiert. Auf diese Weise werden die in Tabelle 2 gelisteten Direktgranulat-Formulierungen E und F erhalten.

**Table 2: Zusammensetzung zinkhaltiger oraler Direktgranulat-Formulierungen (Angaben in %)**

| **Bestandteile** | **E** | **F** |
|---|---|---|
| Ascorbinsäure Retardpartikel (entsprechend ca. 70 % Vit. C) | 44,64 | 41,91 |
| Ascorbinsäure (nicht retardiert) | --- | 10,67 |
| Zinkhaltige Vormischung (entsprechend ca. 6,7 % Zn) | 4,69 | 4,00 |
| Sorbitol | 31,17 | 29,12 |
| Xylitol | 12,50 | 10,67 |
| Sucralose | 0,06 | 0,07 |
| Carmellose-Natrium | 0,75 | 0,64 |
| Calciumcarbonat | --- | 0,53 |
| Zitronensäure | 2,06 | --- |
| Mononatriumcitrat | 1,38 | --- |
| Magnesiumdicitrat (wasserfrei) | 1,88 | 1,60 |
| Aromen | 0,75 | 0,69 |
| Magnesiumstearat | 0,13 | 0,11 |

## Patentansprüche

1. Feste pharmazeutische Zusammensetzung in Form eines oralen Direktgranulats mit retardierter Wirkstofffreisetzung, enthaltend:
(a) Retardpartikel mit einem Kern aus kristalliner Ascorbinsäure und einem Überzug aus einem Lipid mit einem Schmelzpunkt von mindestens 50 °C und optional einem oder mehreren weiteren Hilfsstoffen, wobei das Gewichtsverhältnis zwischen Kern und Überzug zwischen 50:50 und 90:10 liegt, wobei die Lipide im Überzug mindestens 80 Gew.-% eines einfachen Triglycerids enthalten, optional ein einfaches Triglycerid ausgewählt aus der Gruppe von Glyceroltripalmitat, Glyceroltristearat und Glyceroltriarachidat; und
(b) neben den Retardpartikeln zumindest einen wasserlöslichen Hilfsstoff, mit dem die Retardpartikel vermischt werden, wobei die einen oder mehrere wasserlösliche Hilfsstoffe ausgewählt sind aus der Gruppe der Zucker, Zuckeralkohole und Oligosaccharide; sowie
(c) optional einen oder mehrere weitere Hilfsstoffe,
wobei ein oder mehrere Hilfsstoffe gemäß (b) oder (c) optional in granulierter Form vorliegen.

2. Pharmazeutische Zusammensetzung nach Anspruch 1, wobei das Gewichtsverhältnis zwischen Kern und Überzug zwischen 60:40 und 80:20 liegt.

3. Pharmazeutische Zusammensetzung nach den Ansprüchen 1 bis 2, wobei der Überzug zu mindestens 90 Gew.-% aus Glyceroltripalmitat und/oder Glyceroltristearat besteht.

4. Pharmazeutische Zusammensetzung nach den Ansprüchen 1 bis 3, wobei die Stärke des Überzugs so gewählt ist, dass die Zusammensetzung innerhalb von 4 Stunden nicht mehr als 70 % der in ihr enthaltenen Ascorbinsäure freisetzt, gemessen in einer Freisetzungs-Apparatur mit Blattrührer (USP Dissolution Apparatus 2) in 900 mL demineralisiertem Wasser bei 37 °C und einer Rührgeschwindigkeit von 75 UPM.

5. Pharmazeutische Zusammensetzung nach den Ansprüchen 1 bis 4, wobei die Retardpartikel eine gewichtsmittlere Teilchengröße zwischen 100 µm und 500 µm aufweisen.

6. Pharmazeutische Zusammensetzung nach den Ansprüchen 1 bis 5, wobei die Retardpartikel zwischen 30 und 70 Gew.-% der Zusammensetzung ausmachen.

7. Pharmazeutische Zusammensetzung nach den Ansprüchen 1 bis 6, wobei die Hilfsstoffe gemäß (b) und (c) mindestens 60 Gew.-%, bevorzugt mindestens 75 Gew.-%, eines in Wasser löslichen Zuckers oder Zuckeralkohols enthalten.

8. Pharmazeutische Zusammensetzung nach Anspruch 7, wobei der in Wasser lösliche Zucker oder Zuckeralkohol ausgewählt ist aus Saccharose, Maltitol, Mannitol, Sorbitol, Xylitol, und Mischungen derselben.

9. Pharmazeutische Zusammensetzung nach den Ansprüchen 1 bis 8, wobei die Hilfsstoffe gemäß (c) Zitronensäure, Mononatriumcitrat und/oder Dinatriumcitrat enthalten.

10. Pharmazeutische Zusammensetzung nach den Ansprüchen 1 bis 9, wobei die Hilfsstoffe gemäß (b) und (c) nicht mehr als 5 Gew.-% von in Wasser schwerlöslichen Hilfsstoffen enthalten.

11. Pharmazeutische Zusammensetzung nach den Ansprüchen 1 bis 10, wobei Einzeldosen der Zusammensetzung in Stickpacks, Sachets, Glasampullen oder Plastikampullen verpackt sind.

12. Verfahren zur Herstellung einer festen pharmazeutischen Zusammensetzung nach den Ansprüchen 1 bis 11, enthaltend die folgenden Schritte:
(a) Bereitstellen von kristalliner Ascorbinsäure;
(b) Bereitstellen eines geschmolzenen Überzugsmaterials enthaltend ein Lipid mit einem Schmelzpunkt von mindestens 50 °C und optional einen oder mehrere weitere Hilfsstoffe;
(c) Fluidisieren der kristallinen Ascorbinsäure;
(d) Besprühen der fluidisierten kristallinen Ascorbinsäure mit dem geschmolzenen Überzugsmaterial;
(e) Abkühlen der überzogenen Ascorbinsäure, so dass das Lipid erstarrt und Retardpartikel erhalten werden; und
(f) Mischen der so erhaltenen Retardpartikel mit einem oder mehreren wasserlöslichen Hilfsstoffen und optional weiteren Hilfsstoffen.

13. Verfahren nach Anspruch 12, wobei in Schritt (d) das Gewichtsverhältnis zwischen der fluidisierten kristallinen Ascorbinsäure und dem geschmolzenen Überzugsmaterial zwischen 60:40 und 80:20 liegt.

14. Verfahren nach den Ansprüchen 12 bis 13, wobei das Überzugsmaterial zu mindestens 90 Gew.-% aus Glyceroltripalmitat und/oder Glyceroltristearat besteht.

15. Feste pharmazeutische Zusammensetzung in Form eines oralen Direktgranulats mit retardierter Wirkstofffreisetzung, hergestellt durch das Verfahren nach den Ansprüchen 12 bis 14.

## Claims

1. A solid pharmaceutical composition in the form of direct oral granules with extended drug release, comprising:
(a) extended-release particles having a core composed of crystalline ascorbic acid and a coating composed of a lipid whose melting point is at least 50 °C and optionally of one or more further excipients, wherein the weight ratio between core and coating is between 50:50 and 90:10, and wherein the lipids in the coating comprise at least 80 wt% of a simple triglyceride, optionally a simple triglyceride selected from the group of glycerol tripalmitate, glycerol tristearate and glycerol triarachidate; and
(b) besides the extended-release particles, at least one water-soluble excipient with which the extended-release particles are mixed, the one or more water-soluble excipients being selected from the group of sugars, sugar alcohols and oligosaccharides; as well as
(c) optionally one or more further excipients,
wherein one or more excipients under (b) or (c) are optionally present in granulated form.

2. The pharmaceutical composition according to Claim 1, wherein the weight ratio between core and coating is between 60:40 and 80:20.

3. The pharmaceutical composition according to Claims 1 and 2, wherein the coating consists to an extent of at least 90 wt% of glycerol tripalmitate and/or glycerol tristearate.

4. The pharmaceutical composition according to Claims 1 to 3, wherein the thickness of the coating is chosen such that the composition releases not more than 70 % of the ascorbic acid contained therein within four hours, measured in a paddle-stirrer release apparatus (USP Dissolution Apparatus 2) in 900 mL of demineralized water at 37 °C at a stirring speed of 75 rpm.

5. The pharmaceutical composition according to Claims 1 to 4, wherein the extended-release particles have a weight-averaged mean particle size of between 100 µm and 500 µm.

6. The pharmaceutical composition according to Claims 1 to 5, wherein the extended-release particles account for between 30 and 70 wt% of the composition.

7. The pharmaceutical composition according to Claims 1 to 6, wherein the excipients under (b) and (c) comprise at least 60 wt%, preferably at least 75 wt%, of a water-soluble sugar or sugar alcohol.

8. The pharmaceutical composition according to Claim 7, wherein the water-soluble sugar or sugar alcohol is selected from sucrose, maltitol, mannitol, sorbitol, xylitol, and mixtures thereof.

9. The pharmaceutical composition according to Claims 1 to 8, wherein the excipients under (c) comprise citric acid, monosodium citrate and/or disodium citrate.

10. The pharmaceutical composition according to Claims 1 to 9, wherein the excipients under (b) and (c) contain not more than 5 wt% of excipients which are sparingly soluble in water.

11. The pharmaceutical composition according to Claims 1 to 10, wherein individual doses of the composition are packaged in stick packs, sachets, glass ampoules or plastic ampoules.

12. A method for producing a solid pharmaceutical composition according to Claims 1 to 11, comprising the following steps:
(a) providing crystalline ascorbic acid;
(b) providing a molten coating material comprising a lipid with a melting point of at least 50 °C and optionally one or more further excipients;
(c) fluidizing the crystalline ascorbic acid;
(d) spraying the molten coating material onto the fluidized crystalline ascorbic acid;
(e) cooling the coated ascorbic acid, so that the lipid solidifies and extended-release particles are obtained; and
(f) mixing the resultant extended-release particles with one or more water-soluble excipients and optionally further excipients.

13. The method according to Claim 12, wherein the weight ratio between the fluidized crystalline ascorbic acid and the molten coating material in step (d) is between 60:40 and 80:20.

14. The method according to Claims 12 and 13, wherein the coating material consists to an extent of at least 90 wt% of glycerol tripalmitate and/or glycerol tristearate.

15. A solid pharmaceutical composition in the form of direct oral granules with extended drug release, prepared by the method according to Claims 12 to 14.

## Revendications

1. Composition pharmaceutique solide sous la forme d'un granulat direct oral à libération retardée de l'agent actif, contenant:
(a) des particules à effet retard comprenant un noyau en acide ascorbique cristallin et un revêtement en un lipide ayant un point de fusion d'au moins 50 °C et éventuellement un ou plusieurs adjuvants supplémentaires, le rapport en poids entre le noyau et le revêtement étant compris entre 50:50 et 90:10, les lipides dans le revêtement contenant au moins 80 % en poids d'un triglycéride simple, éventuellement un triglycéride simple choisi dans le groupe constitué par le tripalmitate de glycérol, le tristéarate de glycérol et le triarachidate de glycérol ; et
(b) en plus des particules à effet retard, au moins un adjuvant soluble dans l'eau, avec lequel les particules à effet retard sont mélangées, le ou les adjuvants solubles dans l'eau étant choisis dans le groupe constitué par les sucres, les alcools de sucres et les oligosaccharides ; et
(c) éventuellement un ou plusieurs adjuvants supplémentaires,
un ou plusieurs adjuvants selon (b) ou (c) se présentant éventuellement sous forme granulée.

2. Composition pharmaceutique selon la revendication 1, dans laquelle le rapport en poids entre le noyau et le revêtement est compris entre 60:40 et 80:20.

3. Composition pharmaceutique selon les revendications 1 à 2, dans laquelle le revêtement est constitué par au moins 90 % en poids de tripalmitate de glycérol et/ou de tristéarate de glycérol.

4. Composition pharmaceutique selon les revendications 1 à 3, dans laquelle l'épaisseur du revêtement est choisie de telle sorte que la composition ne libère pas plus de 70 % de l'acide ascorbique contenu dans celle-ci en l'espace de 4 heures, mesuré dans un appareil de libération avec agitateur à pale (USP Dissolution Apparatus 2) dans 900 mL d'eau déminéralisée à 37 °C et à une vitesse d'agitation de 75 tours/minute.

5. Composition pharmaceutique selon les revendications 1 à 4, dans laquelle les particules à effet retard présentent une taille de particule moyenne en poids comprise entre 100 µm et 500 µm.

6. Composition pharmaceutique selon les revendications 1 à 5, dans laquelle les particules à effet retard représentent entre 30 et 70 % en poids de la composition.

7. Composition pharmaceutique selon les revendications 1 à 6, dans laquelle les adjuvants selon (b) et (c) contiennent au moins 60 % en poids, de préférence au moins 75 % en poids, d'un sucre ou d'un alcool de sucre soluble dans l'eau.

8. Composition pharmaceutique selon la revendication 7, dans laquelle le sucre ou l'alcool de sucre soluble dans l'eau est choisi parmi le saccharose, le maltitol, le mannitol, le sorbitol, le xylitol et leurs mélanges.

9. Composition pharmaceutique selon les revendications 1 à 8, dans laquelle les adjuvants selon (c) contiennent de l'acide citrique, du citrate de monosodium et/ou du citrate de disodium.

10. Composition pharmaceutique selon les revendications 1 à 9, dans laquelle les adjuvants selon (b) et (c) ne contiennent pas plus de 5 % en poids d'adjuvants difficilement solubles dans l'eau.

11. Composition pharmaceutique selon les revendications 1 à 10, dans laquelle des doses individuelles de la composition sont emballées dans des bâtons, des sachets, des ampoules en verres ou des ampoules en plastique.

12. Procédé de fabrication d'une composition pharmaceutique solide selon les revendications 1 à 11, contenant les étapes suivantes:
(a) la préparation d'acide ascorbique cristallin ;
(b) la préparation d'un matériau de revêtement fondu contenant un lipide ayant un point de fusion d'au moins 50 °C et éventuellement un ou plusieurs adjuvants supplémentaires ;
(c) la fluidisation de l'acide ascorbique cristallin ;
(d) la pulvérisation de l'acide ascorbique cristallin fluidisé avec le matériau de revêtement fondu ;
(e) le refroidissement de l'acide ascorbique revêtu, de telle sorte que le lipide durcisse et que des particules à effet retard soient obtenues ; et
(f) le mélange des particules à effet retard ainsi obtenues avec un ou plusieurs adjuvants solubles dans l'eau et éventuellement des adjuvants supplémentaires.

13. Procédé selon la revendication 12, dans lequel, à l'étape (d), le rapport en poids entre l'acide ascorbique cristallin fluidisé et le matériau de revêtement fondu est compris entre 60:40 et 80:20.

14. Procédé selon les revendications 12 à 13, dans lequel le matériau de revêtement est constitué par au moins 90 % en poids de tripalmitate de glycérol et/ou de tristéarate de glycérol.

15. Composition pharmaceutique solide sous la forme d'un granulat direct oral à libération retardée de l'agent actif, fabriquée par le procédé selon les revendications 12 à 14.
